# FASCICULE DE BREVET EUROPEEN

(11) **EP 2 441 816 B1**
(45) Date de publication et mention de la délivrance du brevet: **29.03.2017**
(21) Numéro de dépôt: 11290420.6
(22) Date de dépôt: 19.09.2011
(51) Int. Cl.: C10G 11/18

(54) **Procédé de craquage catalytique adapté au traitement de charges à faible carbon conradson comportant le recyclage d'une coupe cokante selon une technologie nouvelle**
Katalytisches Krackverfahren für die Behandlung von Rohstoffen mit geringem Conradson-Kohlenstoffanteil mit Wiederaufbereitung eines verkokenden Schnitts nach einer neuen Technologie
Catalytic cracking method suitable for processing feedstocks with low Conradson carbon residue, which comprises recycling a coking cut according to a novel technology

(30) Priorité: 14.10.2010 FR 1004046
(43) Date de publication de la demande: 18.04.2012
(73) Titulaire: IFP Énergies nouvelles, 92852 Rueil-Malmaison Cedex (FR)
(72) Inventeur: Feugnet, Frederic, 69004 Lyon (FR); Roux, Romain, 92500 Rueil Malmaison (FR)

(56) Documents cités:
- WO-A1-93/22400
- GB-A- 1 285 791
- US-A- 4 042 489
- US-A- 5 098 554

## Description

### DOMAINE DEL'INVENTION

La présente invention se situe dans le domaine du craquage catalytique de coupes pétrolières, plus particulièrement de coupes présentant un faible niveau de carbon conradson et une forte teneur en hydrogène et qui, de ce fait, rendent difficile l'obtention du bilan thermique de l'unité.

Dans une unité de craquage catalytique (notée FCC), le bilan thermique est assuré par la combustion du coke déposé sur le catalyseur pendant l'étape de réaction. Cette combustion a lieu dans la zone de régénération. Typiquement, le catalyseur entre dans la zone de régénération avec une teneur en coke (définie comme la masse de coke sur la masse de catalyseur) comprise entre 0,5 et 1 et ressort de la dite zone avec une teneur en coke inférieure à 0,01.

La teneur en carbon conradson (noté CCR en abrégé) de la charge (défini par la norme ASTM D 482) fournit une évaluation de la production de coke au cours du craquage catalytique. En fonction de la teneur en carbon conradson de la charge, le rendement en coke nécessite un dimensionnement spécifique de l'unité pour satisfaire le bilan thermique.

Les coupes lourdes conventionnelles traitées dans une unité de FCC ont généralement des carbon conradson compris dans la fourchette 0,2 à 10%.

Les coupes traitées dans une unité de FCC selon la présente invention peuvent avoir des carbon conradson inférieurs à 0,1 et des teneurs en hydrogène supérieures à 12,7%.

### EXAMEN DE L'ART ANTERIEUR

Il est connu de l'art antérieur de recycler au niveau du régénérateur une coupe issue du FCC à fort potentiel de coke, dite coupe cokante qui est généralement la coupe "slurry", c'est à dire une coupe 360°C+ à dominante aromatique, ou toute coupe hydrocarbonée telle que du Fioul N°2 ou du Fuel domestique. Ce recyclage d'une coupe "slurry" ou d'une coupe fuel N°2 au régénérateur est problématique car, compte tenu des températures régnant dans le régénérateur, de l'ordre de 650°C à 750°C, une partie de ce recycle se vaporise formant des gaz craqués qui vont se retrouver dans la phase diluée du régénérateur en risquant ainsi de créer des points chauds dommageables au bon fonctionnement de l'unité. Ce phénomène souvent appelé "afterburning", peut se définir comme une reprise de combustion en un point non souhaité de l'unité, notamment en entrée de cyclone. On conservera dans la suite du texte le terme d"'afterburning" bien admis et pratiqué par l'homme du métier. GB1285791A décrit un procédé de production d'essence par craquage catalytique d'une coupe hydrocarbure lourde avec un catalyseur régénéré en lit fluidisé comprenant le craquage de la coupe cycle oil légère (LCO) préalablement hydrotraitée dans une phase dense de catalyseur partiellement désactivé provenant du craquage en phase diluée.

Par ailleurs, ce flux de recycle risque de bruler dans le lit de catalyseur en formant localement un front de flamme à haute température qui fait subir au catalyseur de fortes températures locales (points chauds). Ces fortes températures locales combinées à la présence de vapeur d'eau fragilise la partie active du catalyseur (zéolite) et désactive ainsi sa fonction craquante. La présente invention décrit une nouvelle localisation pour effectuer le recyclage de la coupe cokante qui présente plusieurs avantages, dont celui d'éviter la formation de points chauds.

### DESCRIPTION SOMMAIRE DE LA FIGURE

La figure 1 est un schéma du procédé selon l'invention dans lequel on montre le recyclage d'une coupe cokante on une première variante de l'invention.
La figure 2 est un schéma du procédé selon l'invention dans lequel on montre le recyclage d'une coupe cokante selon une seconde variante de l'invention.
La figure 3 est un schéma du procédé selon l'invention dans lequel on montre le recyclage d'une coupe cokante selon une troisième variante de l'invention.

### DESCRIPTION SOMMAIRE DE L'INVENTION

La présente invention s'applique aussi bien à des unités de FCC utilisant un réacteur fonctionnant à courant ascendant (appelé "riser" dans la terminologie anglo-saxonne), qu'à des unités utilisant un réacteur fonctionnant à courant descendant (appelé "downer" dans la terminologie anglo-saxonne).

La présente invention s'applique également à des unités FCC fonctionnant avec un seul réacteur (en écoulement ascendant ou en écoulement descendant), et à des unités FCC fonctionnant avec deux réacteurs. Dans la suite du texte, on parlera de réacteur principal noté (1) pour désigner le réacteur orienté vers la production d'essence, et de réacteur secondaire pour désigner le réacteur dédié à la production de propylène.

Généralement, lorsque les unités FCC fonctionnent avec deux réacteurs, un principal et un secondaire, ces réacteurs sont à écoulement ascendant, mais une unité qui ferait appel à deux réacteurs à écoulement descendant reste dans le cadre de la présente invention.

Typiquement, le riser principal fonctionne avec un rapport catalyseur sur charge compris entre 4 et 15, et préférentiellement compris entre 5 et 10, et avec des températures de sortie riser (notée TS) comprises entre 480°C et 580°C, et préférentiellement comprises entre 500°C et 560°C.

Les conditions optimales de production de propylène dans le riser secondaire sont obtenues pour des températures de sortie dudit riser secondaire comprises entre 550°C et 650°C, préférentiellement entre 580°C et 610°C, des temps de contact compris entre 20 ms et 500 ms, préférentiellement compris entre 50 ms et 200 ms (ms désigne l'abréviation de milliseconde = 10⁻³ seconde), et des flux de solide compris entre 150 et 600 kg/s/m2.

Le temps de contact est défini comme le rapport du volume de catalyseur présent dans le réacteur sur le débit volumétrique de fluide traversant le réacteur aux conditions de mise en oeuvre réactionnelle.

L'ensemble de ces conditions conduit à opérer le riser secondaire à des ratios catalyseur sur charge (noté C/O) compris entre 8 et 35, et préférentiellement compris entre 10 et 25.

Les charges que peut traiter une unité de FCC selon la présente invention sont des charges à carbon conradson inférieur à 0,1 et ayant une teneur en hydrogène supérieure à 12,7%.

Parmi ce type de charge on peut citer :
- les purges d'unité d'hydrocraqueur, dénommées "bleed" dans la terminologie anglo-saxonne, présentant une teneur en hydrogène supérieure à 13%,
- les charges VGO (abréviation de gasoil sous vide) prétraitées sévèrement, ayant un point d'ébullition supérieur à 350°C, et présentant des teneurs en hydrogène supérieur à 12,7%,
- les huiles végétales.

Ces charges peuvent être traitée seules ou en mélange

La présente invention peut se décrire comme un procédé de production d'essence faisant appel à une unité de craquage catalytique possédant au moins un réacteur principal fonctionnant à courant ascendant (appelé "riser") ou à courant descendant (appelé "downer"), et une zone de régénération du catalyseur coké, la dite unité traitant une charge à carbon conradson inférieur à 0,1 et ayant une teneur en hydrogène supérieure à 12,7%, dans laquelle on pratique un recyclage d'une coupe dite cokante, telle par exemple que les coupes suivantes:
- LCO (abréviation de light cycle oil) d'intervalle de distillation compris typiquement entre 220 et 360°C,
- HCO (abréviation de "heavy cycle oil") d'intervalle de distillation compris typiquement entre 360 et 440°C,
- "slurry" d'intervalle de distillation supérieur à 360°C (noté 360°+),
- ou un mélange desdites coupes,

a) Dans une première variante de l'invention, celle ci peut se définir comme un procédé de production d'essence faisant appel à une unité de craquage catalytique (FCC) possédant au moins un réacteur principal (1) fonctionnant à courant ascendant ou à courant descendant, le catalyseur coké en sortie du réacteur (1) étant introduit dans une zone de stripage, appelé stripeur, fonctionnant en lit fluidisé et présentant une phase dense (3) surmontée d'une phase diluée (2), la dite unité traitant une charge lourde à carbon conradson inférieur à 0,1 et à teneur en hydrogène supérieure à 12,7 % poids, procédé dans lequel un recyclage d'une des coupes suivantes LCO, HCO ou "slurry", ou d'un mélange quelconque desdites coupes, dite coupe cokante, est effectué dans une capacité latérale fluidisée (7) placée en dérivation du stripeur, c'est à dire le long d'une ligne de transfert (6), (11), la portion supérieure de la ligne de transfert (6) ayant son origine en un point supérieur de la phase dense (3) du stripeur et la portion inférieure de la ligne de transfert (11) ayant son retour dans la phase dense (3) dudit stripeur en un point situé au dessous du point supérieur, la dite capacité latérale (7) étant placée en amont d'une vanne de réglage (12) du débit de catalyseur, placée sur la portion inférieure de la ligne de transfert (11), et étant munie d'une ligne d'évent (13) permettant le retour des gaz produits dans la phase diluée (2) du stripeur, ladite capacité latérale (7) comprenant en outre dans sa partie inférieure un garnissage inférieur (9) situé en dessous du point d'introduction du flux de recycle (14), et dans sa partie supérieure un garnissage supérieur (8) situé au dessus du point d'introduction du catalyseur par la ligne de transfert (6), procédé dans lequel le flux de soutirage du catalyseur introduit dans la capacité latérale (7) est compris entre 50 et 100 kg/m2/s et le temps de résidence globale dudit catalyseur dans la capacité latérale (7) est compris entre 20 et 100 secondes.
   Dans une autre configuration particulière de la première variante, le catalyseur prélevé dans la phase dense (3) du stripeur et amené dans la capacité latérale (7), est introduit dans la phase diluée de ladite capacité latérale (7) au moyen d'un dispositif de dispersion.
b) Dans une seconde variante de la présente invention, celle ci peut se définir comme un procédé de production d'essence faisant appel à une unité de craquage catalytique (FCC) possédant au moins un réacteur principal (1) fonctionnant à courant ascendant (appelé "riser") ou à courant descendant (appelé "downer"), le catalyseur coké en sortie du réacteur étant introduit dans une zone de stripage, appelé stripeur, fonctionnant en lit fluidisé et présentant une phase dense (3) surmontée d'une phase diluée (2), la dite unité traitant une charge lourde à carbon conradson inférieur à 0,1 et à teneur en hydrogène supérieure à 12,7 % poids, procédé dans lequel un recyclage d'une des coupes suivantes LCO, HCO ou "slurry", ou d'un mélange quelconque desdites coupes, dite coupe cokante, est effectué dans une enceinte tubulaire (17) placée à l'intérieur du stripeur, l'extrémité supérieure de la dite enceinte tubulaire (17) débouchant dans la phase diluée (2) du stripeur, et l'extrémité inférieure de la dite enceinte (17) débouchant dans la phase dense (3) du stripeur.
   Dans une configuration préférée de la seconde variante de l'invention, l'enceinte tubulaire (17) est positionnée de telle façon que la partie immergée dans la phase dense (3) du stripeur représente entre 30 % et 100% de la longueur totale de la dite enceinte tubulaire.
c) Dans une troisième variante de la présente invention, celle ci peut se définir comme un procédé de production d'essence faisant appel à une unité de craquage catalytique (FCC) possédant au moins un réacteur principal (1) fonctionnant à courant ascendant (appelé "riser") ou à courant descendant (appelé "downer"), le catalyseur coké en sortie du réacteur étant introduit dans une zone de stripage, appelé stripeur, fonctionnant en lit fluidisé et présentant une phase dense (3) surmontée d'une phase diluée (2), la dite unité traitant une charge lourde à carbon conradson inférieur à 0,1 et à teneur en hydrogène supérieure à 12,7 % poids, procédé dans lequel un recyclage d'une des coupes suivantes LCO, HCO ou "slurry", ou d'un mélange quelconque desdites coupes, dite coupe cokante, est effectué au sein de la phase dense (3) du stripeur dans une enceinte tubulaire (17') immergée dans ladite phase dense (3), entre deux niveaux de garnissage, un garnissage inférieur et un garnissage supérieur.

La présente invention dans toutes ses variantes est compatible avec un procédé de production d'essence et de coproduction de propylène faisant appel à une unité de craquage catalytique possédant un riser principal (1) et un riser secondaire fonctionnant en parallèle du riser principal et travaillant à des conditions opératoires plus sévères que celles du riser principal, le dit riser secondaire traitant en mélange une coupe oléfinique C4 C5 et/ou une coupe essence et/ ou un oligomerat C5, C6 , C7 ou C8 recyclés.
Dans un tel procédé de craquage catalytique à deux risers, la température de sortie du riser principal (1) est généralement comprise entre 480°C et 580°C, préférentiellement comprise entre 500°C et 560°C, et le rapport C/O est généralement compris entre 4 et 15, et préférentiellement compris entre 5 et 10.

Dans un tel procédé de craquage catalytique à deux risers, la température de sortie du riser secondaire est généralement comprise entre 550°C et 650°C, préférentiellement comprise entre 580°C et 610°C, et le temps de contact est généralement compris entre 20 et 500 ms (milliseconde), préférentiellement compris entre 50 ms et 200 ms.
Enfin, dans le cadre de la présente invention, la coupe cokante de recycle peut également contenir en partie une coupe extérieure à l'unité FCC de type :
- biomasse du type bois ou cellulose
- produit hydrocarboné liquide venant du pétrole
- charbon broyé
- coupe riche en asphalte provenant d'une unité de désalphatage
- cire provenant d'une unité de liquéfaction du charbon par voie indirecte (GTL)
- coke de pétrole
- ou un mélange desdites coupes

Le but principal de la présente invention est d'éviter de créer des points chauds au niveau de la zone de régénération du catalyseur, comme peut le produire un recycle directement effectué dans le régénérateur (selon l'art antérieur), ces points chauds ayant pour effet de désactiver le catalyseur. Un autre objectif de la présente invention est d'éviter le phénomène d"'afterburning" précédemment décrit.

### DESCRIPTION DETAILLEE DE L'INVENTION

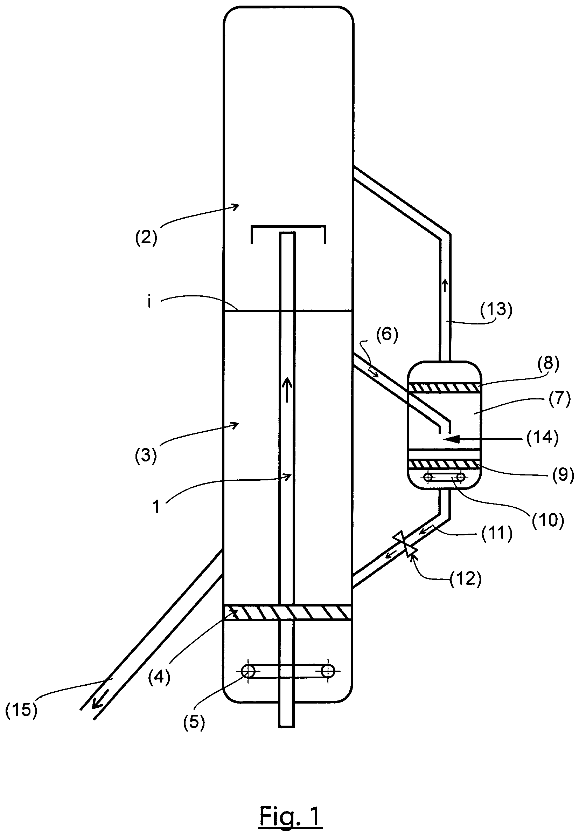

La description détaillée est faite au moyen des figures 1 , 2 et 3 qui représentent la partie réactionnelle d'une unité de FCC.

Les trois figures 1, 2 et 3 ont en commun la structure classique de la partie réactionnelle d'une unité de FCC à savoir un riser (1) dont la partie supérieure est enfermée dans un stripeur comportant une phase dense (3) et une phase diluée (2). Le stripeur est fluidisé par un organe de fluidisation (5), et un garnissage interne(4) est généralement installé dans la partie inférieure de la phase dense (3) du stripeur de manière à réduire les entrainements de particules solides vers la phase diluée (2).

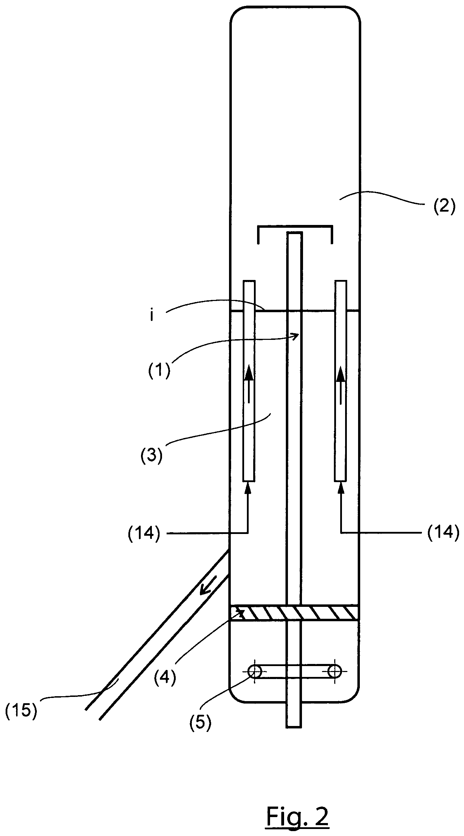

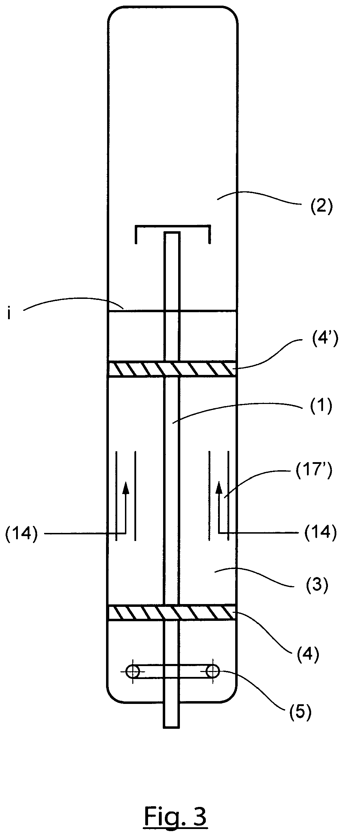

La ligne de transfert (15) permet de conduire le catalyseur coké depuis la phase dense (3) du stripeur vers la zone de régénération (non représentée sur les figures 1, 2 et 3).
a) Selon la première variante de la présente invention, représentée sur la Figure 1, le catalyseur coké est soutiré de la partie supérieure de la phase dense du stripper (3) au moyen d'une ligne de transfert (6), et est envoyé dans une capacité latérale (7) opérée en lit fluidisé, appelée plus loin capacité latérale (7). Cette capacité latérale est située le long d'une ligne de transfert (6,11) du catalyseur allant du point de prélèvement du catalyseur vers le point de réintroduction du catalyseur dans la partie inférieure de la phase dense du stripeur (3) au moyen de la ligne de transfert (11). La capacité latérale fluidisée (7) est située en amont de la vanne de réglage (12) du débit de catalyseur.
   Le flux de recycle est mis en contact avec le catalyseur dans la phase diluée de la capacité latérale (7) de manière à réaliser un bon contactage dudit recycle avec le catalyseur assurant ainsi un dépôt bien homogène de coke additionnel sur le catalyseur. On entend par contactage le processus de mise en contact du flux de recycle et du catalyseur avoisinant. Le catalyseur étant à l'état fluidisé, ce processus de contactage s'étend progressivement à l'ensemble du catalyseur contenu dans la capacité latérale fluidisée (7).
   L'homogénéité du dépôt de coke ainsi obtenu est nettement meilleure que dans le cas où le recycle serait introduit directement dans le lit fluidisé du stripeur ou du régénérateur.
   Le flux de recycle (14) peut être injecté via un ou plusieurs injecteurs. Afin de faciliter la vaporisation de ce recycle, de la vapeur dite de dilution pourra être mise en oeuvre comme cela se fait pour les injecteurs de charge.
   Dans cette première variante, le flux de recycle (14) réagit avec le catalyseur chaud en formant des gaz et du coke dans la capacité latérale fluidisée (7).
   Les gaz issus du craquage du flux de recycle sont renvoyés par une ligne d'évent (13) dans la phase diluée (2) du stripper évitant ainsi qu'ils ne soient envoyés dans le régénérateur.
   Par cet agencement, l"'afterburning" et les risques de points chauds sont évités dans le régénérateur.
   Le catalyseur coké dans la capacité latérale (7) est strippé par de la vapeur, par exemple via un anneau de fluidisation (10), ce qui permet de le débarrasser des hydrocarbures volatils qui sont également renvoyés vers la phase diluée (2) du stripeur par la ligne d'évent (13).
   Un garnissage inférieur (9) peut être mise en place dans la partie inférieure de la capacité latérale (7) afin de limiter l'entraînement de bulles de gaz avec le solide dans le stripeur.
   De la même manière, un garnissage supérieur (8) situé dans la phase diluée de la capacité latérale (7), au dessus du point d'introduction du catalyseur par la ligne de transfert (6), peut être mis en place afin de limiter l'entraînement de solide avec les gaz et permettre ainsi de préserver la qualité de séparation gaz /particules.
   La vanne de réglage de débit (12) placée sur la ligne de transfert (11) permettant le retour du catalyseur de la capacité latérale (7) vers la phase dense (3) du stripeur, permet de régler le niveau de solide dans ladite capacité. Par conséquent, le débit de solide entrant dans la capacité latérale (7) est réglé par l'ajustement du niveau de solide dans le stripper.
   Le dimensionnement de la capacité latérale (7) est tel que le temps de résidence global du catalyseur passant par la dite capacité et retournant dans le stripeur est approximativement le même que pour la partie de solide non prélevé à savoir un temps total de résidence compris entre 10 et 150 secondes, préférentiellement entre 20 et 100 secondes.
   Le flux de solide dans la capacité latérale fluidisée (7) est limité entre 30 et 150 kg/m2/s , préférentiellement entre 50 et 120 kg/m2/s, afin de limiter l'entraînement des bulles de gaz avec le solide retournant dans le stripeur.
b) Selon une seconde variante de la présente invention, représentée sur la Figure 2, le contactage du catalyseur et du flux de recycle (14) est effectué dans une ou plusieurs capacités tubulaires (17) situées à l'intérieur même du stripeur. Toute autre géométrie telle que, par exemple, une capacité en forme de demi cylindre soudé à la paroi du stripeur peut également être envisagée.
   Cette enceinte tubulaire (17) se situe pour la partie inférieure dans la zone dense (3) du stripper au dessus du "packing" (4), lorsqu'il existe, et pour la partie supérieure dans la zone diluée (2) du stripeur.
   Le flux de recycle (14) associé éventuellement à de la vapeur de dilution est injecté dans la partie inférieure de la enceinte tubulaire (17) via un ou plusieurs injecteurs.
   La vaporisation du flux de recycle (14) va diminuer la densité du solide dans l'enceinte tubulaire (17). La différence de pression entre la partie supérieure de l'enceinte tubulaire (17) qui fonctionne en régime dilué, et la partie inférieure de la capacité (17) qui fonctionne en régime dense, engendre une circulation du solide à l'intérieur de ladite enceinte tubulaire (17). Cette circulation naturelle du solide assure un bon contactage du recycle (14) avec le catalyseur. De la même manière que dans la mise en oeuvre précédente, le volume limité de l' enceinte tubulaire (17) garantit un meilleur contactage que si ce recycle (14) était injecté directement dans le lit fluidisé du stripeur ou du régénérateur.
   Le débit de solide dans ce second agencement dépend uniquement du niveau de solide dans le stripeur, c'est à dire de l'interface entre la phase dense (3) et la phase diluée (2), et peut être réglé par ajustement de ce dernier.
   Le craquage du flux de recycle (14) sur le catalyseur chaud produit du coke et des gaz.
   Les gaz quittent l'enceinte tubulaire (17) par l'extrémité supérieure de ladite capacité et se retrouvent donc dans la phase diluée (2) du stripeur. Aucun gaz issu du craquage du recycle (14) n'est donc envoyé dans le régénérateur, et par conséquent l"'afterburning" et la formation de points chauds dans cette zone sont évités.
   Le stripage du catalyseur coké par le recycle (14) est assuré directement par l'organe de fluidisation (4) du stripeur sans mise en oeuvre additionnelle.
c) Selon une troisième variante de la présente invention, représentée sur la Figure 3, le contactage du catalyseur et du recycle est effectué à l'intérieur même du stripeur sous une couche de garnissage (4'), par exemple du type "packing" ou entre 2 couches de garnissage par exemple du type "packing" (4 et 4').

La vaporisation du recycle va créer des bulles dans le lit de catalyseur. Le rôle de la couche supérieure de garnissage (4') est de briser les bulles. Ceci apporte un avantage double. Premièrement, il permet un contactage optimal entre le catalyseur et les plus petites bulles en augmentant le mélange. Ce meilleur contactage permet d'augmenter la conversion du recycle en coke et en fraction plus légère que le recycle.

Deuxièmement, il permet de distribuer ce gaz de manière la plus uniforme possible et ainsi limiter l'entrainement de catalyseur dans la phase diluée (2) du stripeur. Cet entrainement est habituellement aggravée par l'éclatement de larges bulles de gaz à l'interface des phases dense (3) et diluée (2) du stripeur. La couche supérieure de packing (4') a donc une fonction de mélangeur / contacteur pour favoriser la réaction de craquage mais aussi de limiteur d'entrainement.

Le stripage de la partie de solide coké par le recycle est assuré directement dans le stripeur sans mise en oeuvre additionnelle ou par l'ajout d'un garnissage (4) en dessous de la zone d'injection du recycle pour augmenter le contactage entre la vapeur et le solide et également de limiter l'entraînement de bulles de gaz avec le solide vers le régénérateur.

### EXEMPLE COMPARATIF

Afin d'illustrer l'effet recherché par la présente invention, nous avons considéré un premier exemple dit "cas de base" correspondant à une unité de craquage catalytique (FCC) à un seul "riser" d'une capacité de 60 000 barils par jour, soit 300 tonnes par heure, et traitant une charge correspondant à un mélange purge d'hydrocraqueur (Bleed") et VGO hydrotraité.

Les propriétés principales de la charge sont présentées dans le Tableau 1 ci dessous.

**Tableau 1 : Propriétés principale de la charge**

| Charge | | "bleed"+HDT VGO |
|---|---|---|
| Densité | g/cm³ | 0,8552 |
| Teneur en H2 | %poids | 14,04 |
| Soufre | ppm poids | 170 |
| Azote | ppm poids | 298 |
| CCR | | <0,1 |
| Ni | ppm poids | <2 |
| V | ppm poids | <2 |

Cette unité avec recycle de coupe "slurry" dans le régénérateur est opérée dans les conditions présentées dans le Tableau 2. La structure de rendement associée est fournie dans le
1) Selon l'art antérieur le recyclage de la coupe "slurry" est effectué dans le régénérateur. Il en résulte un craquage de ladite coupe générant des gaz craqués inévitablement formés au contact entre la coupe cokante injectée et le catalyseur chaud du régénérateur. Ces gaz craqués représentent environ 2,1% poids de la charge principale, soit un débit de 6,4 tonnes par heure, et sont une source de points chauds lorsqu'ils sont entrainés dans la phase diluée du régénérateur.
2) Selon l'invention, ci dessous.

**Tableau 2 : Conditions opératoires**

| **Conditions opératoires** | |
|---|---|
| C/O | 8,7 |
| Température sortie riser, °C | 525 |
| delta coke | 0,54 |
| Température du régénérateur | 650°C |

**Tableau 3 : Structure de rendement cas de base**

| **Structure de rendement par rapport à la charge** | **%wt** |
|---|---|
| Gaz secs | 1,91 |
| LPG C3/C4 | 29,11 |
| Essence C5-220°C | 55,83 |
| LCO (220- 360°C) | 5,74 |
| > 360°C | 2,64 |
| coke | 4,77 |

Les rendements des gaz craqués et du coke produit par le craquage du "slurry" dans le régénérateur sont présentés dans le Tableau 3 ci dessous.

**Tableau 3 : Structure de rendement des produits issus du craquage du recycle "slurry" dans le régénérateur**

| **Structure de rendement par rapport au "Slurry" recyclé** | **%wt** |
|---|---|
| Gaz secs | 1,86 |
| LPG C3/C4 | 3,22 |
| Essence C5-220°C | *9,5* |
| LCO (220- 360°C) | 28,77 |
| Slurry > 360°C | 37,14 |
| Coke | 19,51 |

1) Selon l'art antérieur le recyclage de la coupe "slurry" est effectué dans le régénérateur. Il en résulte un craquage de ladite coupe générant des gaz craqués inévitablement formés au contact entre la coupe cokante injectée et le catalyseur chaud du régénérateur. Ces gaz craqués représentent environ 2,1% poids de la charge principale, soit un débit de 6,4 tonnes par heure, et sont une source de points chauds lorsqu'ils sont entrainés dans la phase diluée du régénérateur.
2) Selon l'invention, le recyclage de la coupe "slurry" issue de l'unité même, a lieu dans une capacité latérale fluidisée (7) selon la figure 1, placée sur une ligne de transfert (6) prélevant le catalyseur dans la partie supérieure de la phase dense (3) du stripeur. Le catalyseur coké quitte la capacité latérale fluidisée (7) par une ligne de transfert (11) ramenant ledit catalyseur dans la partie inférieure de la phase dense (3) du stripeur.

La capacité latérale fluidisée (7) est placée en amont de la vanne de réglage (12) du débit de catalyseur et possède une ligne d'évent (13) reliant la phase diluée de la dite capacité avec la phase diluée (2) du stripeur.

Le flux de recycle est mis en contact avec le catalyseur dans la phase diluée de la capacité latérale (7) de manière à réaliser un bon contactage dudit recycle avec le catalyseur assurant ainsi un dépôt bien homogène de coke additionnel sur le catalyseur.

La capacité latérale fluidisée (7) est dimensionnée de manière à assurer un temps de contact global de 70 secondes et un flux de solide de 65 kg/m2/s.

Un "packing" inférieur (9) est situé dans la partie inférieure de la phase dense de la capacité latérale (7).

Un "packing" supérieur (8) est placé dans la phase diluée de la capacité latérale (7).

Le delta coke total du système passe de 0,54 selon l'art antérieur, à 0,61 dans le cas de la présente invention.

La température du régénérateur passe corrélativement de 650°C à 658°C pour la même quantité de "slurry" utilisé, et ce grâce au meilleur contactage du flux de recycle avec le catalyseur selon la présente invention.

Le recycle de la coupe "slurry" en dérivation du stripeur dans la capacité latérale fluidisée (7) permet donc d'assurer pleinement le bilan thermique de l'unité avec un effet particulièrement avantageux au regard de l'art antérieur qui provient à la fois de la localisation du point de recyclage en phase diluée de la capacité fluidisée (7), et de la ligne d'évent (13) qui permet d'éviter le phénomène d"'afterburning" en empêchant les gaz craqués d'être entrainés dans le régénérateur.

## Revendications

1. Procédé de production d'essence faisant appel à une unité de craquage catalytique (FCC) possédant au moins un réacteur principal (1) fonctionnant à courant ascendant ou à courant descendant, le catalyseur coké en sortie du réacteur (1) étant introduit dans une zone de stripage, appelé stripeur, fonctionnant en lit fluidisé et présentant une phase dense (3) surmontée d'une phase diluée (2), la dite unité traitant une charge lourde à carbon conradson inférieur à 0,1 et à teneur en hydrogène supérieure à 12,7 % poids, procédé dans lequel un recyclage (14) d'une des coupes suivantes LCO, HCO ou "slurry", ou d'un mélange quelconque desdites coupes, dite coupe cokante, est effectué dans une capacité latérale fluidisée (7) placée en dérivation du stripeur, ce flux de coupe cokante (14) étant introduit dans la phase diluée de la capacité latérale (7), et le catalyseur étant prélevé depuis le stripeur au moyen d'une ligne de transfert supérieure (6) pour être envoyé dans la capacité latérale fluidisée (7), puis soutiré de ladite capacité latérale (7) et réintroduit dans le stripeur au moyen de la ligne de transfert inférieure (11), la portion supérieure de la ligne de transfert (6) ayant son origine en un point supérieur de la phase dense (3) du stripeur et la portion inférieure de la ligne de transfert (11) ayant son retour dans la phase dense (3) dudit stripeur en un point situé au dessous du point supérieur, la dite capacité latérale (7) étant placée en amont d'une vanne de réglage (12) du débit de catalyseur, placée sur la portion inférieure de la ligne de transfert (11), et étant munie d'une ligne d'évent (13) permettant le retour des gaz produits dans la phase diluée (2) du stripeur, ladite capacité latérale (7) comprenant en outre dans sa partie inférieure un garnissage inférieur (9) situé en dessous du point d'introduction du flux de recycle (14), et dans sa partie supérieure un garnissage supérieur (8) situé au dessus du point d'introduction du catalyseur par la ligne de transfert (6), procédé dans lequel le flux de soutirage du catalyseur introduit dans la capacité latérale (7) est compris entre 50 et 100 kg/m2/s, et le temps de résidence global dudit catalyseur dans la capacité latérale (7) est compris entre 20 et 100 secondes.

2. Procédé de production d'essence faisant appel à une unité de craquage catalytique (FCC) selon la revendication 1, dans lequel le catalyseur prélevé dans la phase dense (3) du stripeur et amené dans la capacité latérale (7) est introduit dans la phase diluée de ladite enceinte au moyen d'un dispositif de dispersion.

3. Procédé de production d'essence faisant appel à une unité de craquage catalytique (FCC) possédant au moins un réacteur principal (1) fonctionnant à courant ascendant ou à courant descendant, le catalyseur coké en sortie du réacteur étant introduit dans une zone de stripage, appelé stripeur, fonctionnant en lit fluidisé et présentant une phase dense (3) surmontée d'une phase diluée (2), la dite unité traitant une charge lourde à carbon conradson inférieur à 0,1 et à teneur en hydrogène supérieure à 12,7 % poids, procédé dans lequel un recyclage d'une des coupes suivantes LCO, HCO ou "slurry", ou un mélange quelconque desdites coupes, dite coupe cokante, est effectué dans une enceinte tubulaire (17) placée à l'intérieur du stripeur, l'extrémité supérieure de la dite enceinte débouchant dans la phase diluée (2) du stripeur, l'extrémité inférieur de la dite enceinte (17) débouchant dans la phase dense (3) du stripeur.

4. Procédé de production d'essence faisant appel à une unité de craquage catalytique (FCC) possédant au moins un réacteur principal (1) fonctionnant à courant ascendant ou à courant descendant, le catalyseur coké en sortie du réacteur (1) étant introduit dans une zone de stripage, appelé stripeur, fonctionnant en lit fluidisé et présentant une phase dense (3) surmontée d'une phase diluée (2), la dite unité traitant une charge lourde à carbon conradson inférieur à 0,1 et à teneur en hydrogène supérieure à 12,7 % poids, procédé dans lequel un recyclage d'une des coupes suivantes LCO, HCO ou "slurry", ou un mélange quelconque desdites coupes, dite coupe cokante, est effectué au sein de la phase dense (3) du stripeur dans une enceinte tubulaire (17') immergée dans ladite phase dense (3), entre deux niveaux de garnissage, un garnissage inférieur et un garnissage supérieur.

5. Procédé de production d'essence faisant appel à une unité de craquage catalytique selon la revendication 3, dans lequel l'enceinte tubulaire (17) est positionnée de telle façon que la partie immergée dans la phase dense (3) du stripeur représente entre 30 % et 100% de la longueur totale de la dite enceinte tubulaire (17).

6. Procédé de production d'essence et de coproduction de propylène faisant appel à une unité de craquage catalytique selon la revendication 1, ou selon la revendication 3 ou selon la revendication 4, possédant un riser principal (1) et un riser secondaire fonctionnant en parallèle du riser principal (1) et travaillant à des conditions opératoires plus sévères que celles du riser principal, le dit riser secondaire traitant en mélange une coupe oléfinique C4 C5 et une coupe essence et/ ou un oligomerat C5, C6 , C7 ou C8 recyclés.

7. Procédé de production d'essence faisant appel à l'unité de craquage catalytique selon la revendication 6, dans lequel la température de sortie du riser principal (1) est comprise entre 480°C et 580°C, préférentiellement comprise entre 500°C et 560°C, et le rapport C/O est compris entre 4 et 15, préférentiellement entre 5 et 10, et dans lequel la température de sortie du riser secondaire est comprise entre 550°C et 650°C, préférentiellement comprise entre 580°C et 610°C, et le temps de contact est compris entre 20 et 500 ms (milliseconde), préférentiellement entre 50 ms et 200 ms.

8. Procédé de production d'essence faisant appel à l'unité de craquage catalytique selon la revendication 1, ou selon la revendication ou selon la revendication 4, dans lequel le recycle de coupe cokante contient également en partie une coupe extérieure à l'unité FCC de type :
- biomasse du type bois ou cellulose
- produit hydrocarboné liquide venant du pétrole
- charbon broyé
- coupe riche en asphalte provenant d'une unité de désalphatage
- cire provenant d'une unité de liquéfaction du charbon par voie indirecte (GTL)
- coke de pétrole
- ou un mélange desdites coupes

## Patentansprüche

1. Verfahren zur Herstellung von Benzin unter Verwendung einer katalytischen Krack-Einheit (FCC) mit mindestens einem Hauptreaktor (1), der mit absteigendem oder aufsteigendem Strom arbeitet, wobei der gekokte Katalysator am Ausgang des Reaktors (1) in eine als Stripper bezeichnete Stripping-Zone eingebracht wird, die im Fließbett arbeitet und eine dichte Phase (3) aufweist, über der eine verdünnte Phase (2) angeordnet ist, wobei die Einheit eine schwere Charge mit einem Conradson-Kohlenstoffgehalt von weniger als 0,1 und mit einem Wasserstoffgehalt von mehr als 12,7 Gew.-% behandelt, bei welchem Verfahren ein Rezyklieren (14) einer der folgenden Fraktionen LCO, HCO oder einer "Aufschlämmung", oder eines beliebigen Gemischs der Fraktionen, bezeichnet als kokende Fraktion, in einem seitlichen fluidisierten Kondensator (7) durchgeführt wird, der im Nebenstrom des Strippers angeordnet ist, wobei dieser Strom (14) der kokenden Fraktion in die verdünnte Phase des seitlichen Kondensators (7) eingebracht wird, und wobei der Katalysator aus dem Stripper über eine obere Transferleitung (6) entnommen wird, um in den seitlichen fluidisierten Kondensator (7) geleitet zu werden, dann aus dem seitlichen Kondensator (7) abgezogen wird und über die untere Transferleitung (11) erneut in den Stripper eingebracht wird, wobei der obere Abschnitt der Transferleitung (6) seinen Ursprung an einem oberen Punkt der dichten Phase (3) des Strippers hat, und der untere Abschnitt der Transferleitung (11) seinen Rücklauf in der dichten Phase (3) des Strippers an einem Punkt hat, der unter dem oberen Punkt angeordnet ist, wobei der seitliche Kondensator (7) stromaufwärts von einem Regelventil (12) des Katalysatordurchsatzes angeordnet ist, an dem unteren Abschnitt der Transferleitung (11) angeordnet ist, und mit einer Entlüftungsleitung (13) ausgestattet ist, die den Rücklauf der in der verdünnten Phase (2) des Strippers erzeugten Gase gestattet, wobei der seitliche Kondensator (7) außerdem in seinem unteren Abschnitt eine untere Auskleidung (9), die unter dem Punkt des Einbringens des Rezyklierstroms (14) angeordnet ist, und in seinem oberen Abschnitt eine obere Auskleidung (8), die über dem Punkt des Einbringens des Katalysators durch die Transferleitung (6) angeordnet ist, umfasst, bei welchem Verfahren der Abzugsstrom des Katalysators, der in den seitlichen Kondensator (7) eingebracht wird, zwischen 50 und 100 kg/m²/s liegt, und die gesamte Verweilzeit des Katalysators in dem seitlichen Kondensator (7) zwischen 20 und 100 Sekunden liegt.

2. Verfahren zur Herstellung von Benzin unter Verwendung einer katalytischen Krack-Einheit (FCC) nach Anspruch 1, wobei der Katalysator, der in der dichten Phase (3) des Strippers entnommen wird und in den seitlichen Kondensator (7) geführt wird, in die verdünnte Phase der Umhüllung mit einer Dispersionsvorrichtung eingebracht wird.

3. Verfahren mit absteigendem oder aufsteigendem Strom zur Herstellung von Benzin unter Verwendung einer katalytischen Krack-Einheit (FCC)mit mindestens einem Hauptreaktor (1), der stromaufwärts oder stromabwärts arbeitet, wobei der gekokte Katalysator am Ausgang des Reaktors (1) in eine als Stripper bezeichnete Stripping-Zone eingebracht wird, die im Fließbett arbeitet und eine dichte Phase (3) aufweist, über der eine verdünnte Phase (2) angeordnet ist, wobei die Einheit eine schwere Charge mit einem Conradson-Kohlenstoffgehalt von weniger als 0,1 und mit einem Wasserstoffgehalt von mehr als 12,7 Gew.-% behandelt, bei welchem Verfahren ein Rezyklieren einer der folgenden Fraktionen LCO, HCO oder einer "Aufschlämmung", oder eines beliebigen Gemischs der Fraktionen, bezeichnet als kokende Fraktion, in einer rohrförmigen Umhüllung (17) durchgeführt wird, die im Inneren des Strippers angeordnet ist, wobei das obere Ende der Umhüllung in die verdünnte Phase (2) des Strippers mündet, wobei das untere Ende der Umhüllung (17) in die dichte Phase (3) des Strippers mündet.

4. Verfahren zur Herstellung von Benzin unter Verwendung einer katalytischen Krack-Einheit (FCC)mit mindestens einem Hauptreaktor (1), der mit absteigendem oder aufsteigendem Strom arbeitet, wobei der gekokte Katalysator am Ausgang des Reaktors (1) in eine als Stripper bezeichnete Stripping-Zone eingebracht wird, die im Fließbett arbeitet und eine dichte Phase (3) aufweist, über der eine verdünnte Phase (2) angeordnet ist, wobei die Einheit eine schwere Charge mit einem Conradson-Kohlenstoffgehalt von weniger als 0,1 und mit einem Wasserstoffgehalt von mehr als 12,7 Gew.-% behandelt, bei welchem Verfahren ein Rezyklieren einer der folgenden Fraktionen LCO, HCO oder einer "Aufschlämmung", oder eines beliebigen Gemischs der Fraktionen, bezeichnet als kokende Fraktion, in der dichten Phase (3) des Strippers in einer rohrförmigen Umhüllung (17') durchgeführt wird, die in die dichte Phase (3) eingetaucht wird, zwischen zwei Auskleidungsniveaus, einer unteren Auskleidung und einer oberen Auskleidung.

5. Verfahren zur Herstellung von Benzin unter Verwendung einer katalytischen Krack-Einheit nach Anspruch 3, wobei die rohrförmige Umhüllung (17) derart positioniert wird, dass der Abschnitt, der in die dichte Phase (3) des Strippers eingetaucht wird, zwischen 30 % und 100 % der Gesamtlänge der rohrförmigen Umhüllung (17) beträgt.

6. Verfahren zur Herstellung von Benzin und zur gleichzeitigen Herstellung von Propylen unter Verwendung einer katalytischen Krack-Einheit nach Anspruch 1 oder nach Anspruch 3 oder nach Anspruch 4, mit einem Haupt-Riser (1) und einem sekundären Riser, der parallel zu dem Haupt-Riser (1) arbeitet, und unter Betriebsbedingungen arbeitet, die extremer sind als jene des Haupt-Risers,wobei der sekundäre Riser ein Gemisch einer Olefinfraktion C4, C5 und einer Benzinfraktion und/oder eines OligomeratsC5, C6, C7 oder C8 behandelt, die rezykliert werden.

7. Verfahren zur Herstellung von Benzin unter Verwendung der katalytischen Krack-Einheit nach Anspruch 6, wobei die Temperatur des Ausgangs des Haupt-Risers (1) zwischen 480°C und 580°C, vorzugsweise zwischen 500°C und 560°C, beträgt, und das Verhältnis C/O zwischen 4 und 15, vorzugsweise zwischen 5 und 10, beträgt, und wobei die Temperatur des Ausgangs des sekundären Risers zwischen 550°C und 650°C, vorzugsweise zwischen 580°C und 610°C, beträgt, und die Kontaktzeit zwischen 20 und 500 ms (Millisekunden), vorzugsweise zwischen 50 ms und 200 ms, beträgt.

8. Verfahren zur Herstellung von Benzin unter Verwendung der katalytischen Krack-Einheit nach Anspruch 1 oder nach Anspruch oder nach Anspruch 4, wobei die Rezyklierung der kokenden Fraktion auch teilweise eine Fraktion extern von der FCC vom Typ enthält:
- Biomasse vom Typ Holz oder Cellulose
- flüssiges Kohlenwasserstoffprodukt, das von Erdöl stammt
- zerkleinerte Kohle
- asphaltreiche Fraktion, die von einer Entasphaltierungseinheit stammen
- Wachs, das von einer Kohleverflüssigungseinheit auf indirektem Weg (GTL) stammt
- Petrolkoks
- oder ein Gemisch der Fraktionen.

## Claims

1. A process for the production of gasoline employing a fluid catalytic cracking unit (FCC) having at least one principal reactor (1) operating in riser or downer mode, the coked catalyst from the reactor (1) outlet being introduced into a stripping zone, termed a stripper, operating in fluidized bed mode and having a dense phase (3) surmounted by a diluted phase (2), said unit processing a heavy cut with a Conradson Carbon of less than 0.1 and a hydrogen content of more than 12.7% by weight, in which process a recycle of one of the following cuts: LCO, HCO or slurry, or any mixture of said cuts, termed the coking cut, is carried out in a fluidized side chamber (7) branching off the stripper, that flux of coking cut(14) being introduced in the dilute phase of the side chamber (7), and the catalyst coming from the striper by mean of an upward transfer line (6), and being sent to the side chamber (7), then drawn off said side chamber (7) and reintroduced into the striper by mean of an downward transfer line (11), the upper portion of the transfer line (6) having its origin at an upper point of the dense phase (3) of the stripper and the lower portion of the transfer line (11) having its return to the dense phase (3) of said stripper at a point located below the upper point, said side chamber (7) being placed upstream of a valve (12) for regulating the flow rate of the catalyst, and being provided with a vent line (13) allowing the gases produced to be returned to the diluted phase (2) of the stripper, said side chamber (7) comprising in addition in its lower part a lower packing (9) located below the introduction point of the recycle flux (14), and in its upper part an upper packing (8) located above the introduction point of the catalyst by mean of the transfer line (6), process in which the withdrawing flux of catalyst introduced in the side chamber (7) is in the range from 50 to 100 kg/m2/s, and the global residence time of said catalyst in the side chamber (7) is in the range from 20 to 100 seconds.

2. A process for the production of gasoline using a fluid catalytic cracking unit (FCC) according to claim 1, in which the catalyst withdrawn from the dense phase (3) of the stripper and supplied to the side chamber (7) is introduced into the diluted phase of said vessel by means of a dispersion device.

3. A process for the production of gasoline employing a fluid catalytic cracking unit (FCC) having at least one principal reactor (1) operating in riser or downer mode, the coked catalyst leaving the reactor outlet being introduced into a stripping zone, termed a stripper, operating in fluidized bed mode and having a dense phase (3) surmounted by a diluted phase (2), said unit processing a heavy cut with a Conradson Carbon of less than 0.1 and a hydrogen content of more than 12.7% by weight, in which process a recycle of one of the following cuts: LCO, HCO or slurry, or any mixture of said cuts, termed the coking cut, is carried out in a tubular vessel (17) placed inside the stripper, the upper end of said vessel (17) opening into the diluted phase (2) of the stripper, and the lower end of said vessel (17) opening into the dense phase (3) of the stripper.

4. A process for the production of gasoline employing a fluid catalytic cracking unit (FCC) having at least one principal reactor (1) operating in riser or downer mode, the coked catalyst leaving the reactor outlet being introduced into a stripping zone, termed a stripper, operating in fluidized bed mode and having a dense phase (3) surmounted by a diluted phase (2), said unit processing a heavy cut with a Conradson Carbon of less than 0.1 and a hydrogen content of more than 12.7% by weight, in which process a recycle of one of the following cuts: LCO, HCO or slurry, or any mixture of said cuts, termed the coking cut, is carried out within the dense phase (3) of the stripper in a tubular vessel (17') immersed in said dense phase (3) between two tiers of packing, a lower packing and an upper packing.

5. A process for the production of gasoline using a fluid catalytic cracking unit according to claim 3, in which the tubular vessel (17) is positioned such that the portion immersed in the dense phase (3) of the stripper represents in the range 30% to 100% of the total length of said tubular vessel (17).

6. A process for the production of gasoline and for the co-production of propylene employing a fluid catalytic cracking unit according to claim 1 or according to claim 3, or according to claim 4, having a principal riser (1) and a secondary riser operating in parallel to the principal riser and operating under more severe operating conditions than those of the principal riser, said secondary riser treating, as a mixture, an olefinic C4 C5 cut and/or a gasoline cut and/or a recycled C5, C6, C7 or C8 cut.

7. A process for the production of gasoline employing a fluid catalytic cracking unit according to claim 6, in which the outlet temperature for the principal riser (1) is in the range 480°C to 580°C, preferably in the range 500°C to 560°C, and the C/O ratio is in the range 4 to 15, preferably in the range 5 to 10, and in which the outlet temperature for the secondary riser is in the range 550°C to 650°C, preferably in the range 580°C to 610°C, and the contact time is in the range 20 to 500 ms [millisecond], preferably in the range 50 ms to 200 ms.

8. A process for the production of gasoline employing a fluid catalytic cracking unit according to claim 1 or claim 3 or claim 4, in which the coking cut recycle also in part contains a cut from outside the FCC unit of the following type:
• biomass of the wood or cellulose type;
• liquid hydrocarbon product originating from oil;
• ground coal;
• asphalt-rich cut deriving from a deasphalting unit;
• wax deriving from an indirect coal liquefaction unit (GTL);
• petroleum coke;
• or a mixture of said cuts.
